# EUROPEAN PATENT SPECIFICATION

(11) **EP 4 122 598 B1**
(45) Date of publication and mention of the grant of the patent: **10.04.2024**
(21) Application number: 21186647.0
(22) Date of filing: 20.07.2021
(51) Int. Cl.: B01L 3/00, C12M 3/06

(54) **A SYSTEM FOR DISSOCIATION OF SOLID BIOPSY AND A METHOD FOR INSPECTION**
SYSTEM ZUR DISSOZIATION EINER FESTEN BIOPSIE UND VERFAHREN ZUR INSPEKTION
SYSTÈME DE DISSOCIATION DE BIOPSIE SOLIDE ET PROCÉDÉ D'INSPECTION

(43) Date of publication of application: 25.01.2023
(73) Proprietor: Max-Planck-Gesellschaft zur Förderung der Wissenschaften e.V., 80539 München (DE)
(72) Inventor: Icha Kubánkova, Markéta, 91054 Erlangen (DE); Soteriou, Despina, 91056 Erlangen (DE); Guck, Jochen Reinhold, 91052 Erlangen (DE); Hofemeier Abu Hattum, Shada, 91052 Erlangen (DE); Dirla, Felix, 65931 Frankfurt am Main (DE)
(74) Representative: Hoffmann Eitle

(56) References cited:
- US-A1- 2014 315 303
- US-A1- 2016 272 934
- US-A1- 2017 355 950
- US-A1- 2019 024 033
- US-A1- 2019 212 233
- DESAI SALIL P ET AL: "Micro-Electrical Impedance Spectroscopy and Identification of Patient-Derived, Dissociated Tumor Cells", IEEE TRANSACTIONS ON NANOBIOSCIENCE, IEEE SERVICE CENTER, PISCATAWAY, NY, US, vol. 18, no. 3, 31 July 2019 (2019-07-31), pages 369-372, XP011732944, ISSN: 1536-1241, DOI: 10.1109/TNB.2019.2920743 [retrieved on 2019-06-27]

## Description

### Technical Field

The present invention relates to a system and a method for analysing tissue.

### Technical Background

Biopsy inspection is one of the most common procedures for investigating tissue and diagnosing possible diseases such as cancer, infection, and inflammation. In such a practice, the biopsies are removed percutaneously, typically using a needle, or surgically. After such a removal, the biopsy is prepared for a pathological inspection where the preparation methods vary according to the required test to be performed by the physician.

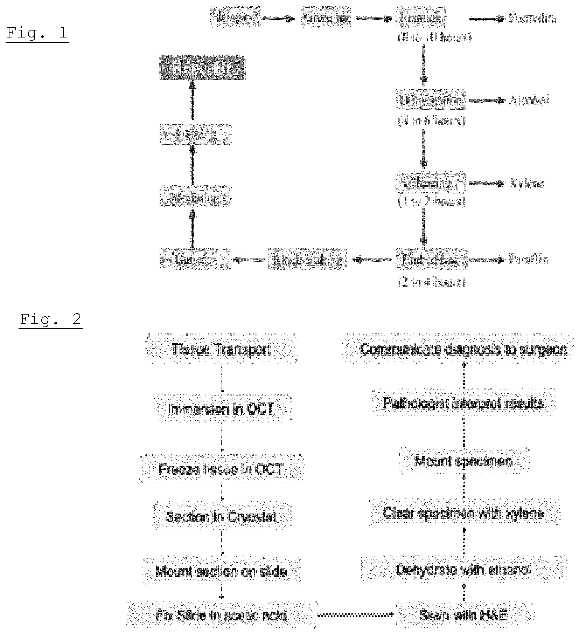

For determining the type or the severity of the disease, a conventional biopsy inspection is performed which is summarized in Figure 1 (which is taken from https://labpedia.net/surgical-pathology-part-1-histopathology-biopsies/) .

As can be seen from that flow chart, after a biopsy has been obtained, a grossing step ensues. Subsequently, using formalin, the sample is fixated. This takes about 8 to 10 hours. Afterwards, a dehydration step using alcohol is performed, which typically takes 4 to 6 hours.

Subsequently, the sample is cleaned using xylene for 1 to 2 hours, and the thus obtained sample is embedded, typically in paraffin, which takes about 2 to 4 hours. From the embedded sample, a block is made, which is then cut and mounted on a slide. Staining steps are performed, and the sample is analysed, typically using a microscope. Based on that analysis, a report is prepared for the physician who can then decide what, if anything, needs to be done in response to the biopsy.

Just looking at the time scales involved in that procedure, it is clear that it would be advantageous if this procedure could be sped up. Further, the time scales prevent intraoperative assessment and delayed treatment in that it will, if a biopsy sample is obtained during a surgery, generally be necessary to interrupt the surgery so as to analyse the sample and to then, if need be, resume it. Accordingly, the resulting burden on the patient is increased, and a treatment is delayed. In particular the latter point can become critical if the future course of treatment needs to be decided as a matter of urgency. In such cases, having the results of the biopsy delayed may prove fatal for a patient.

To speed up the procedure and to allow intraoperative pathological examination (i.e. an examination that can be performed whilst the patient is being operated), a quick freezing of the biopsy sample can shorten the preparation time. In such a case, the biopsy sample is immersed in an optimal cutting temperature (OCT) compound in order to be frozen in a block and then sectioned, as illustrated in Figure 2 (taken from Todd C. Hollon et al., "Near real-time intraoperative brain tumor diagnosis using stimulated Raman histology and deep neural networks", Nature Medicine, 26, 52-58 (2020)). According to that method, the tissue is first transported to the location where it is to be analysed. Subsequently, it is immersed in an OCT solution, where it is frozen. The frozen sample is sectioned in a cryostat and then mounted on a slide. The slide is fixed in acetic acid, then stained with hematoxylin and eosin (H&E) and then dehydrated with ethanol. Subsequently, the specimen is cleared with xylene and mounted. Then, a pathologist interprets the results and communicates the diagnosis to the surgeon, who can then decide how to best proceed.

In the procedure illustrated in Figure 2, since the procedures are to be performed while the operation is still in progress, the pathologist needs to work swiftly on fixing, cleaning, staining and inspecting the biopsy before reporting the results back to the surgeon, thus, in instances, placing him under a significant time pressure. The procedures can range from 10 minutes to several weeks and often rely on the knowledge of the individual medical personal, such as pathologist, haematologist, dermatologist, .... Hence, there is a need for developing a procedure that is faster and less prone to human error.

An additional concern is that it would be desirable if the procedure could be speed up also from the point of view of not wanting to affect the samples that are being collected. There is a concern that if a sample has been removed from the human body for some time, its properties will change, so that any findings obtained using that sample may be less meaningful for the underlying medical condition of the patient being operated. Therefore, it would also be advantageous from the point of view of the accuracy of the diagnosis to speed up the procedure.

US 2017/355950 A1, Salil P. Desai et al., "Micro-Electrical Impedance Spectroscopy and Identification of Patient-Derived, Dissociated Tumor Cells", IEEE Transactions on Nanobioscience, vol. 18, no. 3, US 2014/315303, US 2019/212233, US 2016/272934 and US 2019/024033 are prior art.

### Summary of the Invention

The present invention aims at solving or at least alleviating at least some of the problems mentioned previously.

The invention is defined by the independent claims. Preferred embodiments are set out in the respective dependent claims.

According to claim 1, a system for analysing tissue comprises a dissociation means. A dissociation means is a tool that can be used for dissociating a tissue sample at least partially into individual cells. One such dissociation means is sold under the name "TissueGrinder", which is available from Fast Forward Discoveries GmbH, Germany.

There is furthermore provided a microfluidic flow device through which a fluid comprising the dissociated cells can be made to flow. This flow device could be directly connected to the dissociation means, so that the output of the dissociation means is directly input into the device, or it could be arranged as a separate entity so that the output of the dissociation means can be first retained in some other container or more general holding device before being input into the microfluidic flow device where it can be analysed.

Subsequently, an evaluation device is arranged to evaluate the cells as they are being transported through the microfluidic flow device. Such an evaluation device can, in particular if the cells are being transported through the microfluidic flow device so that individual cells can be analysed, provide important information about the cells at the cellular level, which therefore leads to meaningful results for a surgeon. The evaluation device can analyse based on physical properties such as mechanical properties, cell size, and mass density.

By focusing on individual, dissociated cells, it becomes possible to obtain more meaningful results than could be obtained by an examination of a larger sample. Furthermore, since the microfluidic flow device could be used with partially dissociated tissue samples, which do not need to be prepared using the rather involved procedures mentioned previously, the system is capable of speeding up the analysis of tissue. This applies regardless of whether the dissociated cells are directly input into the microfluidic flow device from the dissociation means or whether they are stored for some time as part of the procedure.

In that context, it is preferred if the system is arranged for a label free analysis of tissue. That is, the individual cells are not subjected to any staining process or any other kind of labelling. With such a label free analysis, it is avoided that the cells to be analysed are changed by the labelling process. Accordingly, such a method has the potential of yielding particularly meaningful results.

It is according to the invention that the evaluation device comprises an imaging means arranged to obtain images of the cells as they are being transported through the microfluidic flow device. Such an optical analysis is particularly useful and has led to particularly good results, also since one can obtain, for example, mechanical properties of the cells by measuring the deformation and by correlating them to the force acting on the cells due to the flow. The imaging means can be supplemented or replaced with means to produce other optical signals such as Brillouin or Raman scattering.

It is preferred that the imaging means comprises an image analysis device that analyses the images obtained of the cells. Accordingly, with such an image analysis device, the data obtained by the imaging device can be analysed. However, it is also possible to only provide the images to a surgeon, who can then make deductions regarding the state of the cells being analysed.

It is preferred that the image analysis device is arranged to analyse the images in real time, that is, as they are being output by the imaging means or with an insignificant delay. This leads to a particularly fast diagnosis result.

Additionally, or alternatively, it is possible that the image analysis device uses a supervised or an unsupervised learning technique, preferably a neuronal network, for analysing the images. Such methods of image analysis have proven to be particularly good ways of analysing complex data such as images. It is also possible to use random forests or PCA as other ways of implementing unsupervised learning.

Alternatively, or additionally, to the imaging means, it is possible that the evaluation device comprises an electrical properties measurement means that is arranged for measuring electrical properties of the cells as they are being transported through the microfluidic flow device. Such an analysis can lead to useful diagnostic information. Preferably, the electrical properties measurement means comprises an impedance measurement means that is arranged for measuring the impedance of the cells as they are being transported through the microfluidic flow device. With such a means, which can be configured to measure the impedance of individual cells, one can obtain useful diagnostic data.

It is preferred if the system comprises a filtering means arranged for filtering out debris caused during the dissociation of the tissue sample. Since debris is removed from a sample to be analysed, the output of the analyses is more meaningful.

It is preferred that the system is arranged so that the individual cells are made to flow through the microfluidic flow device as they are being output by the dissociation means. Accordingly, a significant speed up can be achieved, and it also becomes possible to integrate the system into one single housing, thereby improving the compactness of the system.

It is additionally preferred if the system comprises a means for adding a substance, preferably a medicine, to the dissociated cells prior to the cells being evaluated by the evaluation device. This allows for drug screening or for, more generally, examining how substances affect the cells. Accordingly, one can test whether a certain substance (medicine) could be helpful for treating a certain condition experienced by a patient.

It is preferred if the dissociation means uses an enzymatic and/or a mechanical dissociation method. Such methods are particularly efficient and lead to good results for the dissociated cells.

According to another aspect of the present invention, the invention is defined by the independent method claim. The same explanations set out regarding the device claims apply here. It is worth noting that, preferably, the method can be carried out using the system as described previously.

### Brief Description of the Drawings

Figures 1 and 2 show prior art biopsy methods.
Figure 3 shows a method of analysing a biopsy sample according to an embodiment of the invention.
Figure 4 shows details of an embodiment of Figure 3.
Figures 5 and 6 show analysis results.
Figure 7 shows a schematic depiction of the system according to the first embodiment of the invention.
Figure 8 shows aspects of a second embodiment of the invention.
Figure 9 shows aspects of a third embodiment of the invention.
Figure 10 shows aspects of a fourth embodiment of the invention.

### Detailed Description of the Drawings

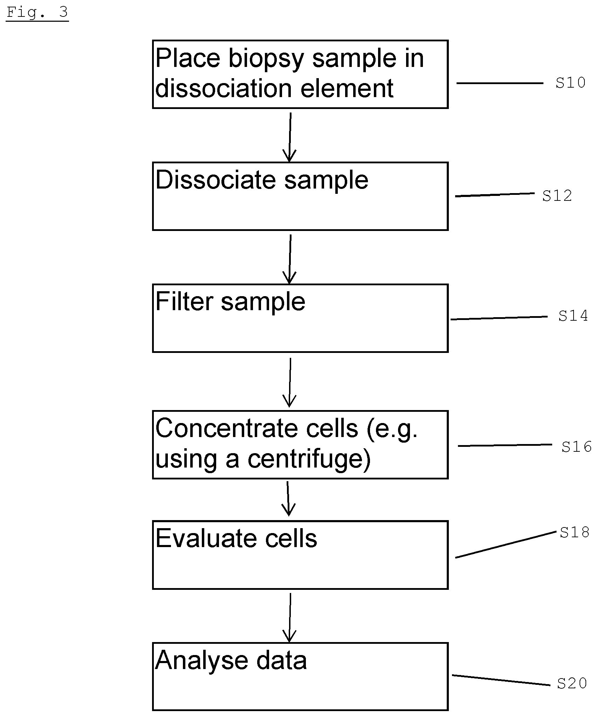

Figure 3 shows steps to be performed in a method according to a first embodiment.

As a first step (step S10), a biopsy sample is placed in a dissociation element which will typically comprise a culture medium.

In that dissociation element, the dissociation of the sample is performed (step S12).

Afterwards (step S14), the medium with the dissociated tissue is made to flow, for example using a pump, through a filter (for example 30-100µm) that allows particles smaller than the specific size, for example 30-100um, to pass.

In a subsequent step (step S16), the filter solution is concentrated, for example by being inserted into a centrifugation tube and then centrifuged. A centrifugal acceleration of, for example 100-500 x g is applied for a few minutes, typically 2-10 minutes, to separate the cells from the culture medium and to resuspend them in the measuring medium which has a higher viscosity than the cell culture medium. It is to be noted that it is not necessary to use a centrifuge, and rather, any process of concentrating the cells can be employed. It is also to be noted that the step of making sure that the cells are suspended in a higher viscosity medium than the cell culture medium is optional.

Subsequently, as shown in step S18, the cells are evaluated. For example, the high viscosity cell suspension is made to flow, for example using a pump, through a tubing into a microfluidic channel where an evaluation step is performed. For example, this evaluation step can be performed by imaging the cell in a constriction area of a microfluidic channel and using different flow rates to vary the hydrodynamic forces on the cells.

Subsequently, in step S20, an analysis of the evaluation of the cells can be performed. If the evaluation includes obtaining images, the images can be analysed for extracting parameters such as the shape or pixel parameters. This can be done in real time or can make use of a post-processing step. From shape parameters mechanical parameters of the cells can be derived, for example from the deviation from a circular shape, in line with the "RAPID" (real-time analysis of physical phenotype in deformational flow)-technology is employed. One example of such a technology is real time deformability cytometry (RT-DC).

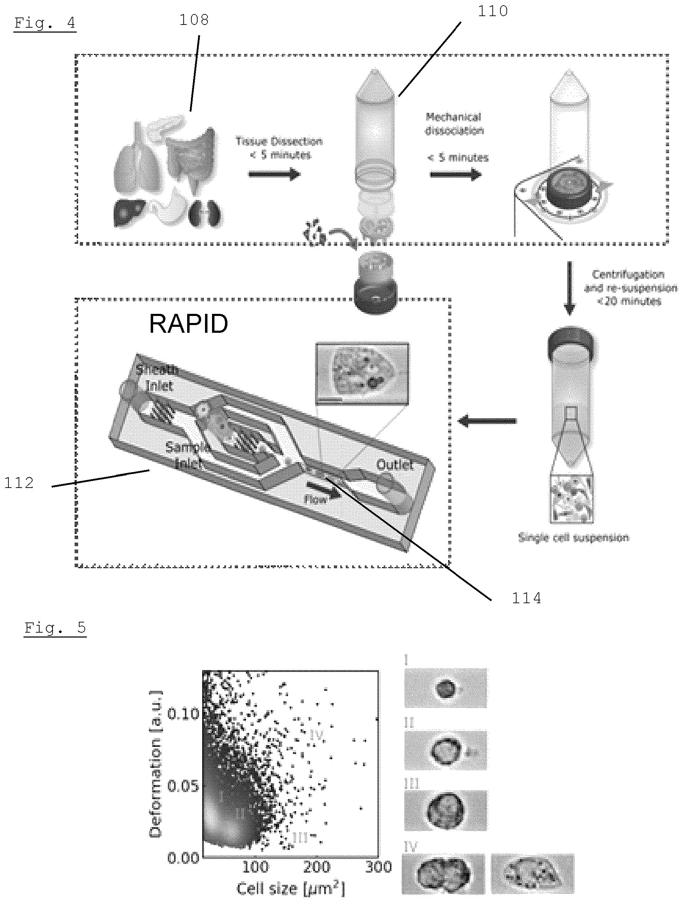

A more detailed embodiment can be seen in Figure 4. In a first step, tissue samples 108 are dissected from organs, which can be extracted during surgery. Subsequently, in a dissociation means 110, the individual cells are dissociated. It is worth noting that, in addition to the mechanical tissue grinder, an alternative dissociation means employing an enzymatic dissociation method can be used. The tissue dissection and the tissue dissociation typically take less than 5 minutes each.

The dissociated sample is centrifuged and resuspended, which typically takes less than 20 minutes, and is then introduced into a tissue analysis means, which can use a RAPID-technology device. Such a technology can rely on the RT-DC functionality that is described in WO 2015/024690 A1.

Using that technology, the suspended cells are introduced into the sheath inlet of the RAPID-device and are then led through a microfluidic channel 114 where an imaging device (not shown) is arranged so as to image the individual cells. Subsequently, the suspended cells are led out using an outlet.

In more detail, a protocol for mechanical dissociation of solid biopsies can be performed as follows:
1. The pathologist extracts a piece of tissue from either a tumour biopsy, a healthy organ or a diseased organ.
2. The biopsy is immediately placed in DMEM (Dulbecco's Modified Eagle's Medium) Advanced medium supplemented with 10% FBS (Foetal Bovine Serum), 1% GlutMAXTM,1% HEPES (2-[4-(2-hydroxyethyl)piperazin-1-yl]ethane-1-sulfonic acid) and 1% penicillin/streptomycin stored at 4°C, processed immediately or frozen in liquid nitrogen for later use. The storage medium is not limited to this formulation.
3. Part of the biopsy is dissected into approximately 2mm (2-4mm) sized pieces and placed into the tissue grinder unit that contains 800-1000µl of DMEM, supplemented with 2% FBS. Approximately 10x 2mm pieces are added per grinding run.
4. The grinding unit is closed using a cell strainer (Mesh size: 100um) and an inverted falcon tube and placed on the TissueGrinder device.
5. A suitable grinding protocol is selected, depending on the solid biopsy. The protocols were pre-optimised to ensure good single cell yield with as little debris as possible. The choice of protocol is tailored to the grinding unit used in the laboratory and will vary if another grinding device is used.
6. Once the protocol has finished the falcon tube is placed upright on a rack, unscrewed and the grinding unit and cell strainer are washed with 5-10ml of DMEM, 2% FBS.
7. The flow through is filtered again through a second cell strainer (Mesh size: 70 um) and the cell suspension is transferred finally into a 15ml falcon tube.
8. Cell suspension is centrifuged at 300 x g for 8 minutes.
9. The supernatant is aspirated, and the cell pellet is resuspended in PBS w/o Ca2+ and Mg2+ supplemented with FBS or Bovine Serum Albumin.
10. The cells suspension is filtered again through a 35um cell strainer and transferred into an Eppendorf tube.
11. The cell suspension is centrifuged at 300 x g for 5 minutes, using a swinging-bucket centrifuge. To stain the cells using a cell surface antibody, the pellet is resuspended in 200µl of the corresponding antibody solution and incubated for 20 minutes at room temperature. The antibodies are washed out by addition of 1ml of PBS containing FBS or BSA (bovine serum albumin) and centrifuged for 5 minutes at 500 x g.
12. The supernatant is removed, and the pellet is diluted in viscous measuring buffer (e.g.: 0.6% (w/v) Methyl Cellulose in Phosphate Saline Buffer without Mg2+ or Ca2+). The filtering process is not limited to the aforementioned mesh sizes which can be varied as needed. It is preferential however to have a sequential filtering from large to small size filters to remove cellular debris.
13. The sample is ready for measuring with the RAPID technology.

RAPD-measurements are performed as previously described (Rosendahl, P., Plak, K., Jacobi, A. et al. Real-time fluorescence and deformability cytometry. Nat Methods 15, 355-358 (2018)) using an AcCellerator instrument (Zellmechanik Dresden GmbH). The steps are set out as follows.
1. The microfluidic chip, made of PDMS bonded on cover glass, consists of a sample inlet, a sheath inlet and an outlet connected by a central channel constriction of either 30 x 30 or 20 x 20um square cross-section and a length of 300 um. The choice of size depends on the sample.
2. The chip is mounted on the stage of an inverted highspeed microscope equipped with a CMOS camera.
3. The chip is pre-filled with measuring buffer before the start of each experiment, using a 1ml Luer-Lok syringe (BD Biosciences) attached to a syringe pump and connected by PEEK-tubing (IDEX Health & Science LLC) which thus provides the sheath fluid.
4. The cell suspension is then drawn into a second 1ml Luer-Lok syringe (BD Biosciences) also attached to the syringe pump which thus provides the sample fluid.
5. The sheath fluid is used to hydrodynamically focus the cells inside the constriction channel. The total flow rate is 0.06 µl/s, of which the sheath flow rate was 0.045 µl/s and the sample flow rate was 0.015 µl/s. The flow rates are not restricted to these values.
6. If fluorescent staining is used, the laser power for each fluorophore is adjusted accordingly, based on single stain controls and an unstained sample. An image of every cell is captured in a region of interest of 250 x 80 pixels at a frame rate of 2,000 fps (it is to be noted that the dimensions of the region of interest and the frame rate are to be chosen as needed). Morphological, mechanical and fluorescent parameters are acquired in real-time.

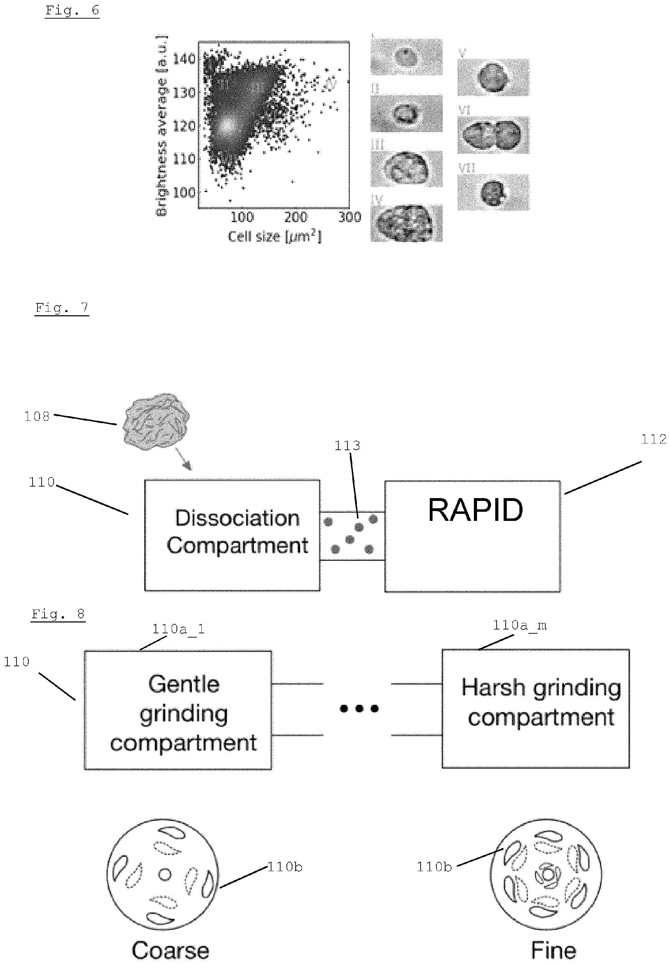

Results of such an analysis can be seen in Figures 5 and 6. For example, from these measurements of the cell deformation, it becomes possible to clearly distinguish between different subpopulations of cells, which allows for a clustering of the cells into groups based on different parameters. In the case at hand, it allows for distinguish different sub-populations of the cells independent of their cell size in terms of their brightness average and their cell size in µm².

Fig. 5 shows that within the leukocyte population of a human kidney, it was possible to identify four different sub-groups based on the cell size and deformation parameter.

Fig. 6 shows that within the cells of a human colon sample, it was possible to identify approximately 6 sub-populations of cells purely based on brightness and size.

Figure 7 shows an overall configuration of the system according to the first embodiment. A biopsy sample 108 is introduced into a dissociation means 110 where individual cells 113 are produced. Subsequently, the output cells 113 are introduced into an analysis means 112, which, in the case at hand, uses the RAPID-technology.

Figure 8 shows details of how the dissociation means can be configured in a second embodiment of the invention. In particular, it is possible to use different grinding compartments, with an initial, more gentle grinding compartment 110a_1 and then one or more harsh grinding compartments 110a_m. As indicated below, this can be implemented by having different grinding vanes 110b that are arranged at different distances relative to each other, where larger distances between the grinding vanes 110b correspond to a gentler grinding. It is to be noted that in all other respects, the second embodiment is identical to the first embodiment described previously.

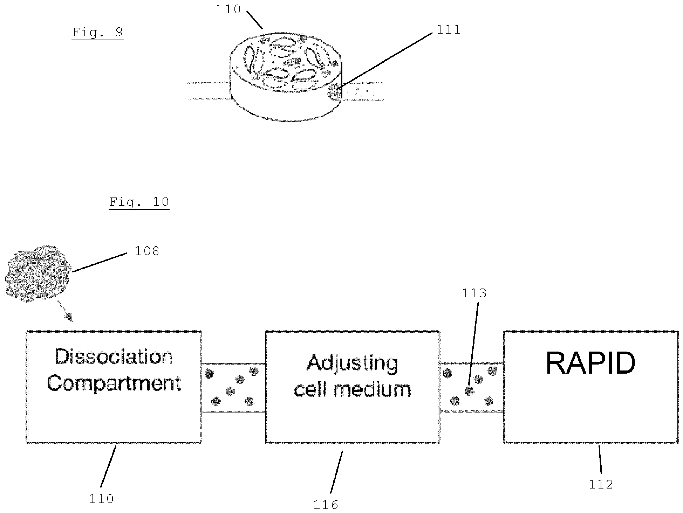

Figure 9 shows the dissociation means 110 according to the third embodiment of the invention. Here, downstream of the actual dissociation means 110, a filter 111 for filtering out debris caused during the dissociation of the tissue sample is arranged. The third embodiment is in all other respects, identical to the first embodiment described previously.

Figure 10 shows a fourth embodiment of the present invention. A biopsy sample 108 is introduced into a dissociation means 110. The individual cells 113 are then transported into an adjusting cell medium 116. In the adjusting cell medium 116, the cell concentration and the viscosity of the carrier fluid 108 is increased, which will avoid centrifugation and resuspension steps. From the adjusting cell medium 116, the individual cells 113 are introduced into the evaluation means 112 that uses, in the present embodiment, the RAPID-technology.

## Claims

1. System for analysing tissue, comprising:
- a dissociation means (110) for at least partially dissociating a tissue sample so as to obtain individual cells,
- a microfluidic flow device (112) through which a fluid comprising the dissociated cells (113) can be made to flow, and
- an evaluation device arranged to evaluate the cells as they are being transported through the microfluidic flow device, the system preferably being arranged for a label free analysis of tissue, the evaluation device comprising an imaging means arranged to obtain images of the cells as they are being transported through the microfluidic flow device.

2. System according to claim 1, the imaging means further comprising an image analysis device arranged for analysing the images obtained of the cells, wherein the image analysis device is more preferably arranged to analyse the images in real time and/or wherein the image analysis device uses a supervised or unsupervised learning technique, preferably a neuronal network, for analysing the images, the image analysis device preferably being arranged for analysing light scattering.

3. System according to claim 1 or 2, the evaluation device comprising an electrical properties measurement means arranged for measuring electrical properties of the cells as they are being transported through the microfluidic flow device, the electrical properties measurement means preferably comprising an impedance measurement means arranged for measuring the impedance of the cells as they are being transported through the microfluidic flow device.

4. System according to one of the preceding claims, the system further comprising a filtering means (111) arranged for filtering out debris created during the dissociation of the tissue sample.

5. System according to one of the preceding claims, the system being arranged so that the individual cells (113) are made to flow through the microfluidic flow device as they are output by the dissociation means.

6. System according to one of the preceding claims, further comprising a means for adding a chemical substance to the dissociated cells prior to the cells being evaluated by the evaluation device.

7. System according to one of the preceding claims, the dissociation means using an enzymatic and/or mechanical dissociation method.

8. Method of analysing tissue, comprising:
- dissociating tissue to be analysed so as to obtain individual cells,
- causing the dissociated cells to flow through a microfluidic channel,
- evaluating the cells as they flow through the microfluidic channel, wherein the evaluation of the cells includes the obtaining of images of the cells as they flow through the microfluidic channel, wherein the method of analysing tissue preferably carries out a label free analysis of tissue, the method preferably being carried out using the device according to one of the preceding claims.

9. Method according to claim 8, , wherein the images are analysed in real time and/or wherein the images are analysed using a neuronal network.

10. Method according to claim 8 or 9, wherein the evaluation comprises measuring electrical properties of the cells as they flow through the microfluidic channel, wherein preferably, the impedance of the cells is measured as the cells flow through the microfluidic channel.

11. Method according to one of claims 8 to 10, further comprising filtering the dissociated cells before they are caused to flow through the microfluidic channel.

12. Method according to one of claims 8 to 11, wherein the dissociated cells are made to flow through the microfluidic channel as they are being dissociated.

13. Method according to one of claims 8 to 12, further comprising a step of adding a chemical substance to the dissociated cells prior to the cells being evaluated.

14. Method according to one of claims 8 to 13, wherein the cells are dissociated using an enzymatic and/or mechanical dissociation method.

15. Use of the system according to one of claims 1 to 7 or the method of one of claims 8 to 14 for screening chemical substances, preferably medicines.

## Patentansprüche

1. System zur Analyse von Gewebe, umfassend:
- ein Dissoziationsmittel (110) zur zumindest teilweisen Dissoziation einer Gewebeprobe, um damit einzelne Zellen zu erhalten,
- eine mikrofluidische Strömungsvorrichtung (112), durch die ein die dissoziierten Zellen (113) umfassendes Fluid zum Strömen gebracht werden kann, und
- eine Auswertungsvorrichtung, die so angeordnet ist, dass sie die Zellen auswertet, während sie durch die mikrofluidische Strömungsvorrichtung transportiert werden, wobei das System vorzugsweise für eine markierungsfreie Analyse von Gewebe angeordnet ist, wobei die Auswertungsvorrichtung eine Abbildungseinrichtung umfasst, die so angeordnet ist, dass sie Bilder der Zellen erhält, während sie durch die mikrofluidische Strömungsvorrichtung transportiert werden.

2. System nach Anspruch 1, wobei die Abbildungseinrichtung weiter eine Bildanalysevorrichtung umfasst, die zur Analyse der von den Zellen erhaltenen Bilder angeordnet ist, wobei die Bildanalysevorrichtung bevorzugter so angeordnet ist, dass sie die Bilder in Echtzeit analysiert und/oder wobei die Bildanalysevorrichtung eine überwachte oder nicht überwachte Lerntechnik, vorzugsweise ein neuronales Netzwerk, zur Analyse der Bilder verwendet, wobei die Bildanalysevorrichtung vorzugsweise zur Analyse von Lichtstreuung angeordnet ist.

3. System nach Anspruch 1 oder 2, wobei die Auswertungsvorrichtung eine Einrichtung zur Messung von elektrischen Eigenschaften umfasst, die zum Messen von elektrischen Eigenschaften der Zellen, während diese durch die mikrofluidische Strömungsvorrichtung transportiert werden, angeordnet ist, wobei die Einrichtung zur Messung von elektrischen Eigenschaften vorzugsweise eine Impedanzmesseinrichtung umfasst, die zur Messung der Impedanz der Zellen während ihres Transports durch die mikrofluidische Strömungsvorrichtung angeordnet ist.

4. System nach einem der vorstehenden Ansprüche, wobei das System weiter eine Filtereinrichtung (111) umfasst, die zum Herausfiltern von Trümmern angeordnet ist, die während der Dissoziation der Gewebeprobe entstehen.

5. System nach einem der vorstehenden Ansprüche, wobei das System so angeordnet ist, dass die einzelnen Zellen (113) durch die mikrofluidische Strömungsvorrichtung zum Strömen gebracht werden, wenn sie von der Dissoziationseinrichtung ausgegeben werden.

6. System nach einem der vorstehenden Ansprüche, weiter umfassend eine Einrichtung zur Zugabe einer chemischen Substanz zu den dissoziierten Zellen, bevor die Zellen durch die Auswertungsvorrichtung ausgewertet werden.

7. System nach einem der vorstehenden Ansprüche, wobei die Dissoziationsmittel ein enzymatisches und/oder mechanisches Dissoziationsverfahren verwenden.

8. Verfahren zur Analyse von Gewebe, umfassend:
- Dissoziation von zu analysierendem Gewebe, um damit einzelne Zellen zu erhalten,
- Strömenlassen der dissoziierten Zellen durch einen mikrofluidischen Kanal,
- Auswerten der Zellen, während sie durch den mikrofluidischen Kanal Strömen, wobei das Auswerten der Zellen das Erhalten von Bildern der Zellen, während sie durch den mikrofluidischen Kanal strömen, einschließt, wobei das Verfahren zur Analyse von Gewebe vorzugsweise eine markierungsfreie Analyse von Gewebe durchführt, wobei das Verfahren vorzugsweise unter Verwendung der Vorrichtung nach einem der vorstehenden Ansprüche durchgeführt wird.

9. Verfahren nach Anspruch 8, wobei die Bilder in Echtzeit analysiert werden und/oder wobei die Bilder unter Verwendung eines neuronalen Netzwerks analysiert werden.

10. Verfahren nach Anspruch 8 oder 9, wobei die Auswertung die Messung von elektrischen Eigenschaften der Zellen umfasst, während sie durch den mikrofluidischen Kanal strömen, wobei vorzugsweise die Impedanz der Zellen gemessen wird, während die Zellen durch den mikrofluidischen Kanal strömen.

11. Verfahren nach einem der Ansprüche 8 bis 10, weiter umfassend das Filtern der dissoziierten Zellen, bevor sie durch den mikrofluidischen Kanal strömengelassen werden.

12. Verfahren nach einem der Ansprüche 8 bis 11, wobei die dissoziierten Zellen durch den mikrofluidischen Kanal strömengelassen werden, während sie dissoziiert werden.

13. Verfahren nach einem der Ansprüche 8 bis 12, weiter umfassend einen Schritt, bei dem den dissoziierten Zellen eine chemische Substanz zugegeben wird, bevor die Zellen ausgewertet werden.

14. Verfahren nach einem der Ansprüche 8 bis 13, wobei die Zellen unter Verwendung eines enzymatischen und/oder mechanischen Dissoziationsverfahrens dissoziiert werden.

15. Verwendung des Systems nach einem der Ansprüche 1 bis 7 oder des Verfahrens nach einem der Ansprüche 8 bis 14 zum Screening von chemischen Substanzen, vorzugsweise von Medikamenten.

## Revendications

1. Système d'analyse de tissu, comprenant :
- un moyen de dissociation (110) pour dissocier au moins partiellement un échantillon de tissu de manière à obtenir des cellules individuelles,
- un dispositif d'écoulement microfluidique (112) à travers lequel un fluide comprenant les cellules dissociées (113) peut s'écouler, et
- un dispositif d'évaluation conçu pour évaluer les cellules pendant qu'elles sont transportées à travers le dispositif d'écoulement microfluidique, le système étant de préférence conçu pour une analyse sans marqueur du tissu, le dispositif d'évaluation comprenant un moyen d'imagerie conçu pour obtenir des images des cellules pendant qu'elles sont transportées à travers le dispositif d'écoulement microfluidique.

2. Système selon la revendication 1, le moyen d'imagerie comprenant en outre un dispositif d'analyse d'image conçu pour analyser les images obtenues des cellules, dans lequel le dispositif d'analyse d'image est plus préférentiellement conçu pour analyser les images en temps réel, et/ou dans lequel le dispositif d'analyse d'image utilise une technique d'apprentissage supervisée ou non supervisée, de préférence un réseau neuronal, pour analyser les images, le dispositif d'analyse d'image étant de préférence conçu pour analyser la diffusion de lumière.

3. Système selon la revendication 1 ou la revendication 2, le dispositif d'évaluation comprenant un moyen de mesure de propriétés électriques conçu pour mesurer les propriétés électriques des cellules pendant qu'elles sont transportées à travers le dispositif d'écoulement microfluidique, le moyen de mesure de propriétés électriques comprenant de préférence un moyen de mesure d'impédance conçu pour mesurer l'impédance des cellules pendant qu'elles sont transportées à travers le dispositif d'écoulement microfluidique.

4. Système selon l'une des revendications précédentes, le système comprenant en outre un moyen de filtrage (111) conçu pour filtrer les débris créés pendant la dissociation de l'échantillon de tissu.

5. Système selon l'une des revendications précédentes, le système étant conçu de telle sorte que les cellules individuelles (113) soient amenées à s'écouler à travers le dispositif d'écoulement microfluidique au fur et à mesure qu'elles sortent du moyen de dissociation.

6. Système selon l'une des revendications précédentes, comprenant en outre un moyen pour ajouter une substance chimique aux cellules dissociées avant que les cellules soient évaluées par le dispositif d'évaluation.

7. Système selon l'une des revendications précédentes, le moyen de dissociation utilisant un procédé de dissociation enzymatique et/ou mécanique.

8. Procédé d'analyse de tissu, comprenant les étapes suivantes :
- la dissociation du tissu à analyser de manière à obtenir des cellules individuelles,
- la mise en écoulement des cellules dissociées à travers un canal microfluidique,
- l'évaluation des cellules pendant qu'elles s'écoulent à travers le canal microfluidique, dans lequel l'évaluation des cellules inclut l'obtention d'images des cellules pendant qu'elles s'écoulent à travers le canal microfluidique, dans lequel le procédé d'analyse de tissu exécute de préférence une analyse sans marqueur du tissu, le procédé étant de préférence exécuté en utilisant le dispositif selon l'une des revendications précédentes.

9. Procédé selon la revendication 8, dans lequel les images sont analysées en temps réel et/ou dans lequel les images sont analysées en utilisant un réseau neuronal.

10. Procédé selon la revendication 8 ou la revendication 9, dans lequel l'évaluation comprend la mesure des propriétés électriques des cellules pendant qu'elles s'écoulent à travers le canal microfluidique, dans lequel, de préférence, l'impédance des cellules est mesurée pendant que les cellules s'écoulent à travers le canal microfluidique.

11. Procédé selon l'une des revendications 8 à 10, comprenant en outre le filtrage des cellules dissociées avant qu'elles soient amenées à s'écouler à travers le canal microfluidique.

12. Procédé selon l'une des revendications 8 à 11, dans lequel les cellules dissociées sont amenées à s'écouler à travers le canal microfluidique au fur et à mesure qu'elles sont dissociées.

13. Procédé selon l'une des revendications 8 à 12, comprenant en outre une étape d'ajout d'une substance chimique aux cellules dissociées avant que les cellules soient évaluées.

14. Procédé selon l'une des revendications 8 à 13, dans lequel les cellules sont dissociées en utilisant un procédé de dissociation enzymatique et/ou mécanique.

15. Utilisation du système selon l'une des revendications 1 à 7 ou du procédé selon l'une des revendications 8 à 14 pour dépister des substances chimiques, de préférence des médicaments.
